# EUROPÄISCHE PATENTSCHRIFT

(11) **EP 2 925 714 B1**
(45) Veröffentlichungstag und Bekanntmachung des Hinweises auf die Patenterteilung: **16.11.2016**
(21) Anmeldenummer: 13798327.6
(22) Anmeldetag: 29.11.2013
(51) Int. Cl.: C07C 45/74

(54) **VERFAHREN ZUR KATALYTISCHEN ALDOLKONDENSATION VON ALDEHYDEN**
METHOD FOR THE CATALYTIC ALDOL CONDENSATION OF ALDEHYDES
PROCÉDÉ D'ALDOLISATION CATALYTIQUE D'ALDÉHYDES

(30) Priorität: 30.11.2012 EP 12195003
(43) Veröffentlichungstag der Anmeldung: 07.10.2015
(73) Patentinhaber: BASF SE, 67056 Ludwigshafen am Rhein (DE)
(72) Erfinder: KROKOSZINSKI, Roland, 67273 Weisenheim a. Berg (DE); OEHLENSCHLÄGER, Steffen, 67098 Bad Dürkheim (DE); PAPP, Rainer, 67346 Speyer (DE); RUDOLPH, Jens, 67549 Worms (DE); ULONSKA, Armin, 67150 Niederkirchen (DE)
(86) Internationale Anmeldenummer: PCT/EP2013/075029
(87) Internationale Veröffentlichungsnummer: WO 2014/083138

(56) Entgegenhaltungen:
- EP-A2- 1 106 596
- WO-A1-2010/105892
- US-A- 2 848 498

## Beschreibung

### HINTERGRUND DER ERFINDUNG

Die vorliegende Erfindung betrifft ein Verfahren zur katalytischen Aldolkondensation von Aldehyden, insbesondere zur Herstellung von α,β-ungesättigten Aldehyden, in einem Mehrphasenreaktor.

### STAND DER TECHNIK

Ungesättigte Aldehyde sind Ausgangsstoffe für die Herstellung einer Vielzahl von organischen Verbindungen und werden in vielen Anwendungen eingesetzt. Sie können u. a. durch Hydrierung zur Herstellung von gesättigten Alkoholen verwendet werden, welche wiederum als Ausgangsstoffe zur Herstellung von Weichmachern, Detergentien oder Lösungsmitteln dienen. Darüber hinaus können die ungesättigten Aldehyde durch Selektivhydrierung in die gesättigten Aldehyde und durch anschließende Oxidation in Carbonsäuren überführt werden.

Technisch werden ungesättigte Aldehyde durch Aldolkondensation von gesättigten Aldehyden unter Wasserabspaltung hergestellt. Bedeutende Aldolkondensationen sind die Umsetzung von n-Butyraldehyd unter Wasserabspaltung zu 2-Ethylhexenal oder die Umsetzung von n-Valeraldehyd unter Wasserabspaltung zu 2-Propylheptenal. Die Einsatzstoffe können neben den linearen Aldehyden auch verzweigte Aldehyde enthalten, die entweder mit sich, mit anderen verzweigten Aldehyden oder mit linearen Aldehyden unter Aldolisierung kondensieren können. Ebenso können Aldehyde mit unterschiedlicher Anzahl von Kohlenstoffatomen unter Aldolreaktion miteinander kondensieren. Die beiden Hydrierprodukte der durch Aldolkondensation gewonnenen Aldehyde 2-Ethylhexenal und 2-Propylheptenal (bzw. deren Isomerengemische) sind 2-Ethylhexanol und 2-Propylheptanol (bzw. deren Isomerengemische) und werden in großem Umfang, u. a. als Weichmacheralkohole, eingesetzt.

Als Katalysator der Aldolkondensation wird im Allgemeinen eine Base, oftmals NaOH, gelöst in Wasser eingesetzt, wobei die wässrige Base neben dem organischen Edukt-/- Produktgemisch eine zweite flüssige Phase bildet. Das während der Reaktion freigesetzte Reaktionswasser reichert sich in der schwereren, wässrigen Phase an. Die organischen Edukte und Produkte bilden die leichtere, organische Phase. Die Reaktionstemperatur der Aldolkondensation liegt typischerweise im Bereich von 80 bis 180 °C. Die Reaktion erfolgt in der Regel unter erhöhtem Druck, der bei Anwesenheit einer Gasphase der Summe der Dampfdrücke von wässriger und organischer Phase entspricht und der typischerweise unterhalb von 10 bar liegt. Während der Reaktion wird Wärme frei, die aus dem Prozess abgeführt werden muss. Die Aldolkondensation läuft typischerweise mit hoher Selektivität (> 95 %) zu den gewünschten Produkten ab. Als wichtige Nebenreaktion erfolgt die Bildung hochsiedender Nebenprodukte. Eine weitere mögliche Nebenreaktion ist die sogenannte Cannizzaro-Reaktion, welche auch zu einem Verbrauch des Katalysators führt.

Da sich der für die Aldolkondensation eingesetzte Katalysator im Wesentlichen in der wässrigen Phase befindet, findet die Reaktion ebenfalls hauptsächlich in der wässrigen Phase statt. Für eine hinreichend schnelle Umsetzung müssen die Edukte daher entsprechend gut in der wässrigen Phase löslich sein. Dies ist für Aldehyde mit mehr als 6 Kohlenstoffatomen nicht mehr der Fall, weshalb hier eine wirtschaftliche Fahrweise ohne Lösemittel und/oder Löslichkeitsvermittler oft nicht mehr möglich ist.

Das für die Aldolkondensation verfügbare Reaktionsvolumen wird über das Volumen der wässrigen Phase festgelegt. Daher ist es wünschenswert, den Reaktor mit einem möglichst hohen Anteil an wässriger Phase zu betreiben. Meistens wird bei kontinuierlicher Reaktionsführung hierfür die aus dem Reaktor austretende wässrige Phase nach der Phasentrennung in den Reaktor zurückgeführt. Eine solche Fahrweise ist z. B. in der EP 1106596 A2 beschrieben. Aufgrund der Verdünnung der wässrigen Phase durch das gebildete Reaktionswasser muss ein Teil der wässrigen Phase ständig aus dem Prozess ausgeschleust und Katalysator muss ergänzt werden.

Um einen ausreichend großen Stofftransport zwischen den beiden flüssigen Phasen zu gewährleisten, sollte darüber hinaus im Reaktor eine entsprechend große Phasengrenzfläche zur Verfügung gestellt werden. Typischerweise wird hierfür über eine Dispergiervorrichtung Mischenergie eingetragen. Diese Mischenergie sollte jedoch nicht zu groß sein, da sonst eine stabile Emulsion der beiden Phasen entstehen kann, die sich nach Austritt des Reaktionsgemischs aus dem Reaktor nicht durch einfache Verfahren, z. B. in einem einfachen Schwerkraftabscheider, vollständig trennen lässt.

In der Vergangenheit wurden unterschiedliche Reaktortypen zur Durchführung der Aldolkondensation von Aldehyden eingesetzt. Die Aldolkondensation kann z. B. in einem gerührten Reaktor, in dem die beiden flüssigen Phasen dispergiert werden, durchgeführt werden. Derartige Verfahren sind z. B. in der DE 927626 und der WO 1993/20034 beschrieben. Nachteilig an diesen Verfahren ist die Verwendung von mechanisch anfälligen drehenden Teilen. Weiterhin ist die Abfuhr der Reaktionswärme über konstruktiv aufwendige interne Wärmetauscherrohre erforderlich.

In der US 5,434,313 wird der Einsatz von drei Mischkreisläufen in Reihe zur Aldolkondensation von n-Butyraldehyd beschrieben. Die Mischenergie wird dabei durch die drei Pumpen der Kreisläufe zur Verfügung gestellt. Zur Erhöhung der Verweilzeit ist je ein Behälter in den zweiten und dritten Kreislauf integriert. Nachteil dieses Reaktionssystems ist der große Aufwand für die Verrohrung und die große erforderliche Anzahl an Wälzpumpen.

In der US 5,434,313 wird weiterhin die Durchführung der Aldolkondensation in einem Rohrreaktor beschrieben. Zur besseren Dispergierung der beiden flüssigen Phasen sind statische Mischelemente oder Füllkörper vorgesehen. Die Wärmeabfuhr soll über die Rohrwand erfolgen. Nachteilig an diesem Konzept ist die große erforderliche Rohrlänge und die aufwendige Art der Wärmeabfuhr. In der EP 1106596 A2 wird ebenfalls der Einsatz eines Rohrreaktors vorgeschlagen, der mit Mischelementen ausgestattet ist. Der Austrag aus dem Rohrreaktor wird einem Phasenscheider zur Trennung der beiden flüssigen Phasen zugeführt. Ein Teil der wässrigen Phase wird gemeinsam mit dem darin gelösten Katalysator in den Reaktor zurückgeführt, der restliche Teil wird ausgeschleust. Durch diese Fahrweise wird im Reaktor ein Überschuss an wässriger Phase eingestellt, die organische Phase liegt dispergiert in der wässrigen Phase vor. Die Reaktionswärme wird aus der rückgeführten wässrigen Phase über einen externen Wärmetauscher abgeführt. Für eine ausreichende Dispergierung der organischen Phase ist eine hohe Strömungsgeschwindigkeit in dem eingesetzten Rohrreaktor erforderlich, die zu einem höheren Druckverlust führt. Vorteilhaft bei diesem Reaktorkonzept ist die rückvermischungsfreie Umsetzung der organischen Edukte. Dies ist jedoch mit mehreren Nachteilen, wie die erforderliche große Rohrlänge und die damit verbundene große Anzahl an Mischelementen verbunden. Außerdem ist ein hoher Energieeintrag zur Dispergierung der organischen Phase erforderlich, die den Einsatz größerer Pumpen und somit einen höheren Stromverbrauch erfordert. Der typische Druckverlust für die angegebenen Füllkörper wie SMV2 von Sulzer und VFF liegt bei 150 mbar/m. Daraus lässt sich für das in der EP 1106596 A2 beschriebene Verfahren ein typischer Leistungseintrag von 50 bis 80 kW/m³ Flüssigvolumen ableiten.

US2848498 beschreibt ein Verfahren zur zweiphasigen Aldolkondensation, wobei die Reaktionsmischung in eine Beruhigungszone fließt und wobei die Reaktionszone turbulent ist.

Der Erfindung liegt die Aufgabe zugrunde, ein verbessertes Verfahren zur katalytischen Aldolkondensation in einem zweiphasigen flüssigen Reaktionsgemisch zur Verfügung zu stellen. Dieses soll sich zur Aldolkondensation von Aldehyden mit hoher Selektivität zum ungesättigten Aldolkondensationsprodukt (ungesättigter Aldehyd) eignen. Insbesondere liegt der Erfindung die Aufgabe zugrunde, ein Verfahren zur katalytischen Aldolkondensation bereitzustellen,
- das eine kompakte Bauweise des Reaktors aufweist,
- bei dem einfach ein großer Anteil an wässriger Phase im Reaktor eingestellt werden kann, ohne dass wässrige Phase aus einem externen Abscheider in den Reaktor rückgeführt werden muss,
- bei dem sich die Wärmeabfuhr einfach gestaltet,
- und bei dem eine ausreichende Dispergierung der organischen Phase mit möglichst geringem Energieeintrag möglich ist.

Überraschenderweise wurde gefunden, dass die gestellte Aufgabe effektiv gelöst werden kann, wenn sowohl die Reaktion als auch die Koaleszenz der beiden flüssigen Phasen in einem Apparat kombiniert werden. Hierfür wird mittels einer langsam nach oben durchströmten, nicht durchmischten Beruhigungszone im oberen Teil des Apparates das Absetzen der schwereren wässrigen Phase ermöglicht. Diese wässrige Phase reichert sich dadurch in der darunter liegenden, durchmischten Reaktionszone an.

### ZUSAMMENFASSUNG DER ERFINDUNG

Gegenstand der Erfindung ist ein Verfahren zur katalytischen Aldolkondensation wenigstens eines Aldehyds in einem zweiphasigen flüssigen Reaktionsgemisch in einem Reaktor, der eine Reaktionszone und eine unmittelbar darüber befindliche Beruhigungszone aufweist, wobei die Reaktionszone rückvermischt ist und wobei man in der Reaktionszone eine in einer kontinuierlichen wässrigen katalysatorhaltigen Phase dispergierte aldehydhaltige Phase erzeugt und einen Strom des zweiphasigen Reaktionsgemischs aus der Reaktionszone in die Beruhigungszone aufsteigen und koaleszieren lässt, wobei sich im oberen Bereich der Beruhigungszone eine kontinuierliche organische Phase ausbildet.

Ein weiterer Gegenstand der Erfindung ist die Verwendung einer Vorrichtung, umfassend einen Reaktor, der eine Reaktionszone und eine unmittelbar darüber befindliche Beruhigungszone aufweist, wobei die Reaktionszone eine Vorrichtung zur Erzeugung einer in einer kontinuierlichen wässrigen Phase dispergierten organischen Phase aufweist und die Beruhigungszone die Koaleszenz des zweiphasigen Reaktionsgemischs und die Ausbildung einer kontinuierlichen organischen Phase im oberen Bereich der Beruhigungszone ermöglicht, zur katalytischen Aldolkondensation wenigstens eines Aldehyds in einem zweiphasigen flüssigen Reaktionsgemischs.

### BESCHREIBUNG DER ERFINDUNG

Der Begriff "wässrige Phase" bezeichnet im Rahmen der Erfindung die Phase, die als Hauptkomponente Wasser enthält. Sofern die im Reaktionsgemisch enthaltenen organischen Verbindungen eine gewisse Wassermischbarkeit aufweisen, kann die wässrige Phase entsprechende Anteile an gelösten organischen Verbindungen enthalten. Dementsprechend bezeichnet der Begriff "organische Phase" im Rahmen der Erfindung die Phase, die als Hauptkomponente organische Verbindungen, wie die zur Aldolkondensation eingesetzten Aldehyde und die Produkte der Aldolkondensation, enthält.

Durch das erfindungsgemäße Verfahren gelingt es, dass sich Reaktionszone und Beruhigungszone in einem einzigen Reaktionsbehälter befinden.

Das erfindungsgemäße Verfahren wird bevorzugt kontinuierlich durchgeführt.

Die wässrige katalysatorhaltige Phase besteht weitgehend aus Wasser. Gewünschtenfalls kann die wässrige Phase zusätzlich wenigstens ein organisches, wassermischbares Lösungsmittel enthalten. Als organisches Lösungsmittel können z. B. Propandiol, Glycerin, Diethylenglycol oder Dimethylformamid eingesetzt werden.

Der Anteil an Wasser und organischem Lösungsmittel an der wässrigen Phase beträgt vorzugsweise wenigstens 60 Gew.-%, besonders bevorzugt wenigstens 80 Gew.-%, bezogen auf das Gesamtgewicht der wässrigen Phase.

In einer bevorzugten Ausführungsform enthält die wässrige Phase keine zugesetzten organischen Lösungsmittel. Nicht zu den zugesetzten organischen Lösungsmitteln im Sinne der Erfindung zählen die in der wässrigen Phase gelösten Anteile an Aldehydausgangsmaterial, Produkten der Aldolkondensation und reaktionstypischen Verunreinigungen. Der Anteil an Wasser an der wässrigen Phase beträgt dann vorzugsweise wenigstens 60 Gew.-%, besonders bevorzugt wenigstens 80 Gew.-%, bezogen auf das Gesamtgewicht der wässrigen Phase.

Optional kann die wässrige Phase Phasentransfer-, oberflächenaktive oder amphiphile Reagenzien oder Tenside enthalten.

Bevorzugte Katalysatoren für das erfindungsgemäße Verfahren sind wasserlösliche, basische Verbindungen wie z. B. Hydroxide, Hydogencarbonate, Carbonate, Carboxylate oder ihre Gemische in Form ihrer Alkali- oder Erdalkaliverbindungen verwendet werden. Vorzugsweise kommen Alkalihydroxide, wie Natriumhydroxid, zum Einsatz.

Die Konzentration des Katalysators in der kontinuierlichen wässrigen Phase in der Reaktionszone liegt vorzugsweise in einem Bereich von 0,1 bis 15 Gew.-%, besonders bevorzugt von 0,2 bis 5 Gew.-%, insbesondere von 1 bis 3 Gew.-%.

Das erfindungsgemäße Verfahren eignet sich für die Umsetzung von Aldehyden oder Aldehydgemischen, die eine Kondensationsreaktion eingehen können. Wird nur ein Aldehyd eingesetzt, so muss dieser zwei α-Wasserstoffatome am gleichen Kohlenstoffatom in Nachbarschaft zur CO-Gruppe besitzen. Werden zwei oder mehrere unterschiedliche Aldehyde eingesetzt, so muss mindestens einer der Aldehyde zwei α-Wasserstoffatome am gleichen Kohlenstoffatom aufweisen.

Geeignete Aldehyde für das erfindungsgemäße Verfahren sind Aldehyde mit 1 bis 15, bevorzugt 3 bis 15, besonders bevorzugt 4 bis 6 Kohlenstoffatomen.

Geeignete Aldehyde mit zwei α-Wasserstoffatomen sind beispielsweise Acetaldehyd, Propanal, n-Butyraldehyd, n-Valeraldehyd, 3-Methylbutyraldehyd, n-Hexanal, 3-Methylpentanal, 4-Methylpentanal, n-Heptanal, n-Octanal, n-Nonanal und n-Decanal. Diese Aldehyde eignen sich auch für eine Homokondensation.

Geeignete Aldehyde mit einem α-Wasserstoffatom sind beispielsweise Isobutyraldehyd, 2-Methylbutyraldehyd, 2-Methylpentanal, 2-Ethylhexanal, Cyclohexylaldehyd.

Bevorzugte Einsatzstoffe für das erfindungsgemäße Verfahren sind: n-Butyraldehyd, n-Valeraldehyd, Gemische aus n-Butyraldehyd und Isobutyraldehyd, Gemische aus n-Valeraldehyd mit 2-Methylbutyraldehyd und/oder 3-Methylbutyraldehyd. Ebenfalls kann ein Gemisch aus C₄- und C₅-Aldehyden eingesetzt werden. Diese Aldehyde können z. B. durch Hydroformylierung von Olefinen hergestellt werden.

Bei Einsatz von mehr als einem Aldehyd oder einem Aldehydgemisch können die Einzelkomponenten getrennt in den Strom der Katalysatorlösung eingespeist werden. Ebenfalls ist es möglich, alle Edukte vor der Einspeisung zu mischen und gemeinsam einzuspeisen. Weiterhin können die Aldehyde als Lösung eingesetzt werden. Als Lösungsmittel können inerte, in der Katalysatorlösung kaum lösliche Flüssigkeiten wie z. B. Kohlenwasserstoffe, wie Pentan, Hexan, Ligroin, Cyclohexan oder Toluol, verwendet werden.

### Reaktionszone

Erfindungsgemäß bildet in der Reaktionszone die wässrige Phase die kontinuierliche Phase des zweiphasigen Reaktionsgemischs. Bevorzugt beträgt der Volumenanteil der wässrigen Phase in der Reaktionszone wenigstens 70 %, besonders bevorzugt wenigstens 80 %, bezogen auf das Gesamtvolumen des zweiphasigen Reaktionsgemischs in der Reaktionszone.

In dem erfindungsgemäßen Verfahren ist der Anteil der wässrigen Phase in der Reaktionszone damit im Allgemeinen wesentlich größer als in einem durchmischten Reaktor des Standes der Technik ohne Beruhigungszone bei gleichem Reaktionsumsatz. In Letzterem würde sich ohne einen extern zugeführten wässrigen Strom in der Reaktionszone eine organische kontinuierliche Phase einstellen, was negative Folgen für Umsatz und Selektivität der Reaktion hätte.

Der Volumenanteil der wässrigen Phase in der Reaktionszone kann unter Anderem durch entsprechende Ausführung der Beruhigungszone (z. B. das Volumen, Art und Umfang etwaiger Einbauten und/oder Füllkörper) so eingestellt werden, dass die wässrige Phase die kontinuierliche Phase in der Reaktionszone darstellt.

Die Reaktionszone ist rückvermischt. Insbesondere wird eine Reaktionszone eingesetzt, die bezüglich der wässrigen Phase und bezüglich der organischen Phase fluiddynamisch rückvermischt (d. h. an jeder Stelle in der Reaktionszone befinden sich nahezu gleiche Konzentrationsverhältnisse bezüglich der wässrigen und der organischen Phase). Das Mischen dient dabei zur makroskopischen Durchmischung der Reaktionszone und zur Dispergierung der organischen Phase zu kleinen Tropfen in der kontinuierlichen wässrigen Phase. Es wurde gefunden, dass sich eine Rückvermischung in der Reaktionszone positiv auf die Selektivität des Prozesses auswirkt. Durch die Rückvermischung ist die stationäre Konzentration an Edukt gering und damit werden weniger hoch siedende Kondensationsprodukte gebildet. Ebenso gestaltet sich die Abfuhr der Reaktionswärme aus einem gewälzten rückvermischten System einfacher, da z. B. mit einem außenliegenden Wärmetauscher gearbeitet werden kann.

In einer speziellen Ausführungsform des erfindungsgemäßen Verfahrens wird zur Rückvermischung wenigstens ein der Reaktionszone zugeführter Strom und/oder wenigstens ein in einem externen Kreislauf geführter Strom aus der Reaktionszone (Wälzstrom) eingesetzt.

Geeignete Mischvorrichtungen sind z. B. dynamische Mischer (d. h. Mischer, deren Mischorgane bewegliche Teile enthalten) und statische Mischer (d. h. Mischer ohne bewegte Teile im Inneren, die insbesondere nach dem Inline-Prinzip arbeiten). Bevorzugt wird wenigstens eine Mischvorrichtung eingesetzt, die ausgewählt ist unter Mischdüsen, Rührern, Mischpumpen, statischen Mischelementen, Füllkörperschüttungen, etc. Geeignete Rührertypen umfassen z. B. Propellerrührer, Impellerrührer, Scheibenrührer, Blattrührer, Ankerrührer, Schrägblattrührer, Kreuzbalkenrührer, Wendelrührer, Schneckenrührer, etc.

Bevorzugt wird zur Erzeugung der in der kontinuierlichen wässrigen katalysatorhaltigen Phase dispergierten aldehydhaltigen Phase in der Reaktionszone wenigstens eine Mischdüse eingesetzt. Dabei erfolgt bevorzugt nur ein geringer Leistungseintrag durch die Mischleistung der Düse. Die in den Reaktor insgesamt eingetragene Mischleistung beträgt vorzugsweise höchstens 0,5 kW pro m³ Flüssigvolumen, besonders bevorzugt höchstens 0,3 kW pro m³ Flüssigvolumen. Hier liegt ein wesentlicher Unterschied zu dem in der EP 1 106 596 beschriebenen Verfahren, in dem, wie bereits ausgeführt, die Mischleistung bei 50 bis 80 kW/m³ und damit um zwei Größenordnungen höher liegt.

Bevorzugt ist die Reaktionszone als Schlaufenreaktor oder als Rührkessel ausgebildet. Geeignete Schlaufenreaktoren sind z. B. Freistrahlreaktoren, Strahlschlaufenreaktoren, Strahldüsenreaktoren, etc.

In einer bevorzugten Ausführungsform ist die Reaktionszone als Freistrahlreaktor ausgeführt. Freistrahlreaktoren und ihre Auslegung sind z. B. in K. H. Tebel, H.-O. May, Chem.-Ing.-Tech. 60 (1988), Nr. 11, S. 912 - 913 und der darin zitierten Literatur beschrieben, worauf hier Bezug genommen wird.

Die Zuführung des Aldehyds erfolgt bevorzugt in der Reaktionszone, im Bereich der hohen lokalen Mischenergie des Mischorgans, zum Beispiel in Nähe der Rührerblätter eines Rührers oder an der Düsenspitze einer Mischdüse. Dadurch wird für eine gute Dispergierung und Einmischung des Aldehyds in der wässrigen Reaktionszone gesorgt.

Besonders vorteilhaft gelingt die Zuführung und Einmischung des Aldehyds, wenn dieser über eine Zweistoffdüse zugegeben wird. Eine Abbildung einer geeigneten Zweistoffdüse findet sich in Ullmanns Encyclopedia of Industrial Chemistry, 5th Edition, Volume B 4, Seite 280, Figure 6, A und wird dort als "two-phase jet nozzle" bezeichnet. In dem zugehörigen Artikel sind Zweiphasenströmungen aus einer Gas- und einer Flüssigphase beschrieben. Die abgebildete Düse kann jedoch in dem erfindungsgemäßen Verfahren für flüssig/flüssig Zweiphasenströmungen eingesetzt werden. Dann wird bevorzugt die wässrige Phase dort eingeführt, wo in der Zeichnung "Liquid" vermerkt ist und die organische Phase wird in den Ringraum geführt, dort wo in der Zeichnung "Gas" vermerkt ist.

Alternativ ist es möglich, den Aldehyd in einen in einem externen Kreislauf geführten Strom aus der Reaktionszone (Wälzstrom) einzuspeisen.

Zusätzlich zum Aldehyd muss auch Katalysator der Reaktionszone zugeführt werden. Bevorzugt erfolgt die Zugabe des Katalysators in einen in einem externen Kreislauf geführten Strom aus der Reaktionszone (Wälzstrom). Bevorzugt erfolgt die Zugabe des Katalysators dann auf der Saugseite (stromaufwärts) der in dem Wälzstrom enthaltenen Fördervorrichtung. Wird der Katalysator auf der Saugseite z. B. einer Wälzpumpe zugegeben, so findet vorteilhaft eine gute Einmischung in der Pumpe statt. Die Zugabe des Katalysators erfolgt im Allgemeinen in Form einer konzentrierten wässrigen Lösung.

In einer bevorzugten Ausführungsform des erfindungsgemäßen Verfahrens ist die Reaktionszone als Freistrahlreaktor ausgestaltet. Dabei wir ein Teil des Reaktionsgemisches der Reaktionszone entnommen und über eine Düse im oberen Bereich der Reaktionszone wieder zugeführt. Bevorzugt erfolgt die Entnahme eines Teils der Reaktionsgemischs im unteren Bereich der Reaktionszone und speziell am bodenseitigen Ende. Hierdurch wird eine Ringströmung in der Reaktionszone erzeugt. Als Düse wird vorzugsweise eine Zweistoffmischdüse eingesetzt. Vorzugsweise erfolgt über die Zweistoffmischdüse die Zuführung des Wälzstroms und die Zuführung des Aldehyds in die Reaktionszone. Die Düse ist vorzugsweise nach unten gerichtet axial in der Reaktionszone angeordnet. In einer bevorzugten Ausführungsform befindet sich am unteren Ende der Reaktionszone eine Prallplatte.

Die bei der Aldolkondensation gebildete Reaktionswärme kann in einer geeigneten Ausführung direkt in der Reaktionszone durch integrierte Wärmeübertrager abgeführt werden. In einer bevorzugten Ausführung wird ein in einen Wälzkreis integrierter externer Wärmeübertrager zur Wärmeabfuhr eingesetzt.

Die Wärmeabfuhr erfolgt in diesem Fall kostengünstig über einen externen Wärmeübertrager, der in den um den Reaktor führenden Wälzkreis integriert ist. Der umgewälzte Strom sorgt vorzugsweise gleichzeitig zur Bereitstellung des Treibstrahls der Düse und somit für die Durchmischung des Apparates. Die Wälzpumpe des externen Wälzkreises saugt überwiegend wässrige Phase aus dem unteren Bereich der Reaktionszone an, wodurch die Gefahr der Bildung einer stabilen Emulsion, die nicht mehr durch einfaches Absetzen zur Koagulation gebracht werden kann, minimiert wird.

### Beruhigungszone

In der Beruhigungszone findet eine Phasenumkehr statt, d. h. die Emulsion aus kontinuierlicher wässriger Phase und disperser organischer Phase in der Reaktionszone kehrt sich zu einer Emulsion aus kontinuierlicher organischer Phase und disperser wässriger Phase in der Beruhigungszone um.

Die Querschnittsfläche der Beruhigungszone muss ausreichend groß ausgeführt sein, so dass eine Phasentrennung stattfinden kann und sich die wässrige Phase im Gegenstrom absetzen kann. Daher kann sich der Durchmesser des Apparateteils, in dem sich die Beruhigungszone befindet, von dem Durchmesser des Apparateteils, in dem sich die Reaktionszone befindet, unterscheiden.

Bevorzugt soll die aufwärts gerichtete Leerrohrgeschwindigkeit der beiden flüssigen Phasen in der Beruhigungszone weniger als 10 mm/s betragen. Besonders bevorzugt soll sie weniger als 5 mm/s betragen. Die Angabe der Geschwindigkeit erfolgt dabei auch bei Einsatz von Einbauten und/oder Füllkörpern berechnet auf das leere Rohr.

In einer bevorzugten Ausführung enthält die Beruhigungszone Einbauten und/oder Füllkörper. Füllkörper können in Form von (geordneten) Packung oder einer Schüttung aus Füllkörpern eingesetzt werden. Somit lässt sich in der Beruhigungszone eine bessere Koaleszenz der beiden Phasen erzielen.

Beispiele für Einbauten sind z. B. Filterplatten, Strombrecher, Kolonnenböden, Lochbleche oder sonstige auch als Mischvorrichtungen eingesetzte Vorrichtungen. Als Einbauten eignen sich auch mehrere enge, parallel geschaltete Rohre unter Ausbildung eines Vielrohrreaktors. Besonders bevorzugt sind strukturierte Mischerpackungen oder Demisterpackungen. Geeignete Füllkörper sind beispielsweise Raschigringe, Sättel, Pallringe, Telleretten, Maschendrahtringe oder Maschendrahtgewebe. Als vorteilhaftes Packungs- bzw. Füllkörpermaterial hat sich Stahl erwiesen, da dieser aufgrund seiner Oberflächeneigenschaften besonders die Koaleszenz fördert.

Bevorzugt wird aus der kontinuierlichen organischen Phase im oberen Bereich der Beruhigungszone ein Austrag entnommen.

Optional kann der Austrag aus der Beruhigungszone in einer geeigneten Ausführungsform einer Aufarbeitung zur Isolierung einer an dem Aldolkondensationsprodukt angereicherten Fraktion unterzogen werden.

Bevorzugt wird der Austrag aus der Beruhigungszone einer Umsetzung in wenigstens einem weiteren Reaktor unterzogen. Somit ist eine weitere Umsatzsteigerung an Aldolkondensationsprodukt(en) möglich. Insbesondere erfolgt die weitere Umsetzung in nur einem weiteren Reaktor.

Bevorzugt wird zur weiteren Umsetzung wenigstens ein Reaktor mit Strömungsrohrcharakteristik eingesetzt, d. h. ein Reaktor, der eine möglichst geringe Rückvermischung in Strömungsrichtung aufweist. In einer bevorzugten Variante ist dieser Reaktor rohrförmig ausgeführt. Um Rückvermischung zu vermeiden und eine größere Oberfläche für die Reaktion zur Verfügung zu stellen, enthält der Reaktor vorzugsweise Einbauten, d. h. geordnete Packungen, z. B. Blech- oder Gewebepackungen, und/oder eine regellose Schüttung von Füllkörpern. Bevorzugt sind Packungen und Füllkörper aus Stahl. Der Reaktor mit Strömungsrohrcharakteristik kann als separate Vorrichtung ausgeführt sein. In einer speziellen Ausführungsform ist der Reaktor mit Strömungsrohrcharakteristik direkt oberhalb des ersten, rückvermischt betriebenen Reaktor angeordnet.

Der zur weiteren Umsetzung eingesetzte Reaktor wird bevorzugt adiabatisch betrieben. In einer bevorzugten Ausführungsform enthält der zur weiteren Umsetzung eingesetzte Reaktor eine geordnete Packung, z. B. Blech- oder Gewebepackungen, Packung und/oder eine regellose Schüttungen von Füllkörpern. Bevorzugt sind Packungen und Füllkörper aus Stahl.

Dieser Begriff "adiabatisch" wird im Rahmen der vorliegenden Erfindung im technischen und nicht im physiko-chemischen Sinne verstanden. Unter adiabatischer Reaktionsführung wird eine Vorgehensweise verstanden, bei der die bei der Reaktion freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem Reaktor abgeführt, abgesehen von einem Restanteil, der durch natürliche Wärmeleitung und Wärmeabstrahlung vom Reaktor an die Umgebung abgegeben wird.

Mit dem erfindungsgemäßen Verfahren kann ein hoher Endumsatz im Reaktionsaustrag erzielt werden. Der Reaktionsaustrag ist dabei der Austrag aus der Beruhigungszone oder, falls vorhanden, dem in Stromrichtung letzten zur Aldolkondensation eingesetzten Reaktor. Bevorzugt wird nach dem erfindungsgemäßen Verfahren ein Umsatz von wenigstens 95 Gew.-%, bevorzugt wenigstens 97 Gew.-%, bezogen auf das Gesamtgewicht des zur Aldolkondensation eingesetzten linearen Aldehyds, erhalten.

Nachdem das Produktgemisch aus der Beruhigungszone bzw. dem Nachreaktor ausgetreten ist, kann es einer Kühlung unterzogen werden, z. B. in einem nachgeschalteten Wärmetauscher.

Der flüssige Reaktionsaustrag wird vorzugsweise in einem Flüssig-Flüssig-Trennbehälter in Katalysatorphase und Produktphase getrennt. Dies kann in Absitzbehältern verschiedener Bauart oder Zentrifugen erfolgen.

Das in der Aldolkondensation gebildete Reaktionswasser verdünnt die Katalysatorlösung und muss daher ständig aus dem Prozess entfernt werden. In dem erfindungsgemäßen Verfahren erfolgt die Entfernung von Wasser vorzugsweise ausschließlich mit dem flüssigen Austrag aus der Beruhigungszone oder, falls vorhanden, dem (in Stromrichtung letzten) Nachreaktor. Die nach flüssig-flüssig-Trennung erhaltene wässrige Katalysatorphase kann als Abwasser aus dem Verfahren ausgeschleust werden. In einer alternativen Ausführung kann die abgetrennte wässrige Katalysatorphase, gegebenenfalls nach Ausschleusung einer kleinen Teilmenge und entsprechendem Ersatz durch frische Katalysatorlösung, auch in die Aldolkondensation zurückgeführt werden.

Das nach Abtrennung der Katalysatorphase erhaltene Produkt kann durch bekannte Verfahren gereinigt werden, z. B. durch Destillation.

Die durch das erfindungsgemäße Verfahren hergestellten Aldolkondensationsprodukte können vorteilhaft zur Herstellung von gesättigten Alkoholen durch Hydrierung verwendet werden. Die so erhaltenen gesättigten Alkohole dienen beispielsweise zur Herstellung von Weichmachern, Detergentien oder Lösungsmitteln. Als Vorstufe für Weichmacheralkohole eignen sich speziell die ungesättigten C₈- und C₁₀-Aldehyde. Weiterhin können die Aldolkondensationsprodukte durch Selektivhydrierung in die gesättigten Aldehyde und diese durch eine anschließende Oxidation in Carbonsäuren überführt, d. h. zur Herstellung von Carbonsäuren verwendet werden. Darüber hinaus werden ungesättigte Aldehyde wegen ihrer Reaktivität bei vielen Synthesen eingesetzt. Ein weiteres Einsatzgebiet von gesättigten und ungesättigten Aldehyden ist die Verwendung als Riechstoff.

### FIGURENBESCHREIBUNG

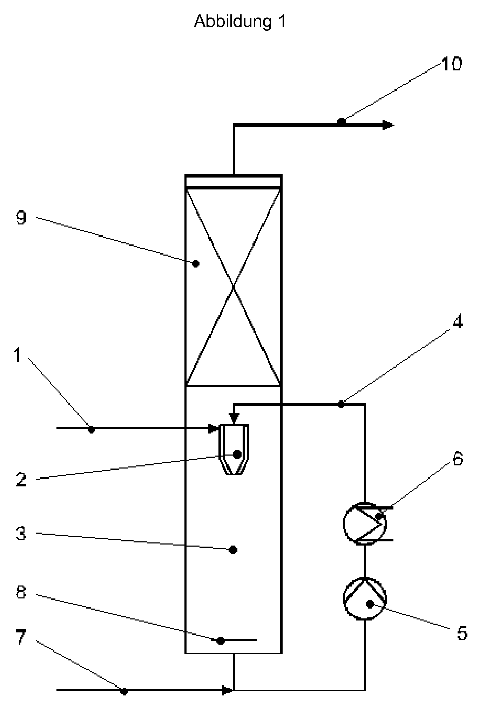

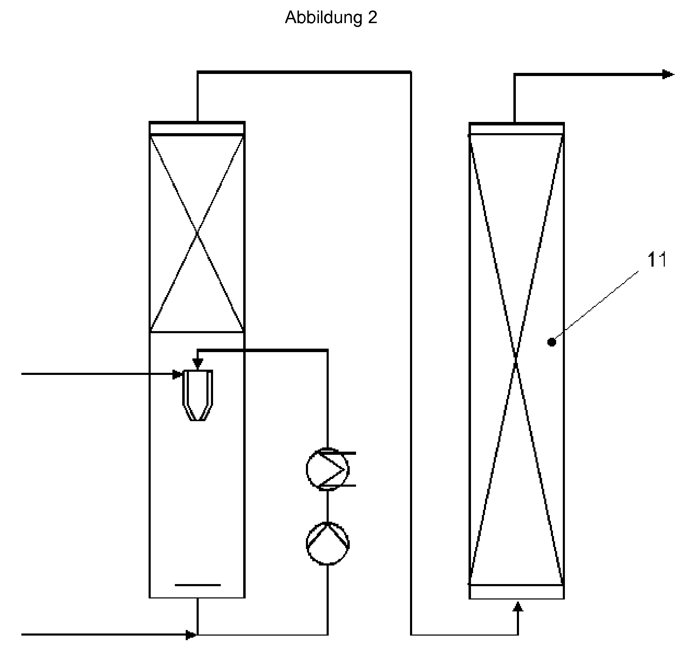

Die Erfindung wird im Folgenden anhand der Figuren 1 und 2 näher erläutert.

### Bezugszeichenliste

(1) Aldehydzuführung
(2) Zweistoffdüse
(3) rückvermischte Reaktionszone
(4) Wälzstrom
(5) Wälzpumpe
(6) externer Wärmetauscher
(7) Zuführung Katalysatorlösung
(8) Prallplatte
(9) Beruhigungszone
(10) Reaktoraustrag
(11) Nachreaktor

Eine mögliche Ausführungsform der Erfindung ist zur Erläuterung in Figur 1 dargestellt. Der Aldehydfeed (1) gelangt über eine Zweistoffdüse (2) in die rückvermischte Reaktionszone (3) des Reaktors. Die Durchmischung der Reaktionszone und die Dispergierung der organischen Phase erfolgt durch den eingetragenen Impuls des Treibstahls der Düse. Als Treibstrahl dient der im unteren Bereich des Reaktors abgezogene Wälzstrom (4), der vorher durch eine Wälzpumpe (5) verdichtet und über einen externen Wärmetauscher (6) zur Abfuhr der Reaktionswärme geführt wird. In den Wälzstrom wird die konzentrierte Katalysatorlösung (7) zugegeben. Eine Prallplatte (8) kann am Reaktorboden vorgesehen werden, um die Abscheidung von organischen Tropfen zu erleichtern. Die Reaktionszone ist überwiegend mit wässriger Phase, in der der gelöste Katalysator vorliegt, gefüllt. Darin sind organische Tropfen, die die Reaktionsedukte und Reaktionsprodukte enthalten, dispergiert. Das Koaleszieren der organischen Tropfen und das Absetzen der wässrigen Phase erfolgt in der Beruhigungszone (9). Diese kann mit einer Packung oder mit Füllkörpern gefüllt sein, welche die Koaleszenz begünstigen. Außerdem findet hier noch Restumsatz statt, d. h. die Beruhigungszone dient auch als Nachreaktor. Insbesondere werden hier langsamer reagierende, verzweigte Aldehydisomere umgesetzt. Innerhalb der Beruhigungszone bzw. am Ausgang des Reaktors findet Phasenumkehr statt, d. h. es bildet sich eine kontinuierliche organische Phase, in der wässrige Tropfen dispergiert vorliegen. Der Reaktoraustrag (10) wird am Kopf des Reaktors abgezogen. Er besteht zum überwiegenden Teil aus organischer Phase. Die wässrige Phase enthält neben dem gelösten Katalysator Reaktionswasser und Wasser, welches über den Katalysatorstrom eingebracht wurde.

Eine weitere mögliche Ausführungsform der Erfindung ist in Figur 2 dargestellt. Dort wird dem Produktaustritt aus der Beruhigungszone ein Nachreaktor (11) nachgeschaltet, um einen höheren Umsatz zu erzielen. Der Nachreaktor kann wie die Beruhigungszone ((9) in Abbildung 1) des Hauptreaktors ebenfalls mit einer Packung oder mit Füllkörpern gefüllt sein, um die axiale Rückvermischung zu minimieren, was zu einem höheren Reaktionsumsatz führt und um die Koaleszenz der beiden Phasen zu begünstigen.

### Beispiel 1:

Es wurde eine Apparatur analog zu Figur 2 eingesetzt. Der erste Reaktor hatte im unteren Teil eine Höhe von 5,6 m und einen Durchmesser von 0,8 m. Auf diesen unteren Teil folgte ein zweiter Teil mit einer Höhe von 2,0 m und einem Durchmesser von 1,1 m. Der untere Teil wurde durch eine Düse durchmischt. Im oberen Teil wurden 1 m³ Füllkörper (Pallringe mit 35 mm ∅ aus V2A-Stahl (1.4541)) eingebracht. Der zweite Reaktor hatte eine Höhe von 16,3 m und einen Durchmesser von 0,5 m und wurde mit 3,0 m³ Füllkörpern (Pallringe mit 35 mm ∅ aus V2A-Stahl (1.4541)) gefüllt. Die Anlage wurde kontinuierlich mit 8,4 t/h einer Aldehyd-mischung (88,8 % n-Valeraldehyd; 9,8 % 2-Methylbutanal; 0,2 % 3-Methylbutanal, Rest gelöste Butane und Butene) beschickt. Über den Umpumpkreislauf wurden 20 t/h gefördert. Die Zweistoffdüse hatte einen Innendurchmesser von 26,7 mm und erzeugte einen Druckverlust von 0,45 bar. Der spezifische Leistungseintrag ergibt sich zu 0,09kW/m³. In der Packung im ersten Reaktor ergibt sich eine Leerrohrgeschwindigkeit von 3,2 mm/s. Die Natronlaugekonzentration in der wässrigen Phase wurde durch Zufuhr von 20 % Natronlauge auf 2,5 Gew.-% gehalten. In den beiden Reaktoren wurde ein Druck von Pe = 6 bar und eine Temperatur von 145 °C eingestellt.

Durch GC-Analysen wurde die Zusammensetzung des Austrages bestimmt.

| Komponente | GC Flächen-% |
|---|---|
| n-Valeraldehyd | 1,4 |
| 2-Methybutanal | 4,9 |
| 3-Methylbutanal | 0,01 |
| 2-Propylheptenal | 81,2 |
| 4-Methyl-2-propylhexenal | 9,7 |
| 5-Methyl-2-propylhexenal | 0,4 |
| Hochsieder | 1,5 |

Die Umsätze der einzelnen Komponenten wurden bestimmt:

| Komponente | Umsatz |
|---|---|
| n-Valeraldehyd | 98,6 % |
| 2-Methybutanal | 55 % |
| 3-Methylbutanal | 95 % |

### Beispiel 2:

Es wurde eine Apparatur analog zu Figur 1 eingesetzt. Der Reaktor hat einen Durchmesser von 1,5 m bei einer Höhe von 14 m. Der untere Teil wurde durch eine Düse durchmischt. Im oberen Teil wurden 8 m strukturierte Packung aus Edelstahl (304L) eingebracht. Die Anlage wurde kontinuierlich mit 25 t/h einer Aldehydmischung (99,85 % n-Butyraldehyd; 0,07 % Isobutyraldehyd, Rest ist gelöstes Propan und Propen) beschickt. Über den Umpumpkreislauf wurden 135 t/h gefördert. Die Zweistoffdüse hatte einen Innendurchmesser von 50 mm und erzeugte einen Druckverlust von 2,2 bar. Der spezifische Leistungseintrag ergibt sich zu 0,75 kW/m³. In der Packung im Reaktor ergibt sich eine Leerrohrgeschwindigkeit von 6 mm/s. Die Natronlaugekonzentration in der wässrigen Phase wurde durch Zufuhr von 25 % Natronlauge auf 4,0 Gew.-% gehalten. Im Reaktor wurde ein Druck von Pe = 2,75 bar und eine Temperatur von 90 °C eingestellt.

Durch GC-Analysen wurde die Zusammensetzung des Austrages bestimmt.

| Komponente | GC Flächen-% |
|---|---|
| n-Butyraldehyd | 0,4 |
| Isobutyraldehyd | 0,01 |
| 2-Ethylhexenal | 96,9 |
| 4-Methyl-2-Ethylpentenal | 0,15 |
| Hochsieder | 2,2 |

Die Umsätze der einzelnen Komponenten wurden wie folgt bestimmt:

| Komponente | Umsatz |
|---|---|
| n-Butyraldehyd | 96,4 % |
| Isobutyraldehyd | 86 % |

## Patentansprüche

1. Verfahren zur katalytischen Aldolkondensation wenigstens eines Aldehyds in einem zweiphasigen flüssigen Reaktionsgemisch in einem Reaktor, der eine Reaktionszone und eine unmittelbar darüber befindliche Beruhigungszone aufweist, wobei die Reaktionszone rückvermischt ist und wobei man in der Reaktionszone eine in einer kontinuierlichen wässrigen katalysatorhaltigen Phase dispergierte aldehydhaltige Phase erzeugt und einen Strom des zweiphasigen Reaktionsgemischs aus der Reaktionszone in die Beruhigungszone aufsteigen und koaleszieren lässt, wobei sich im oberen Bereich der Beruhigungszone eine kontinuierliche organische Phase ausbildet.

2. Verfahren nach Anspruch 1, wobei sich die Reaktionszone und die Beruhigungszone in einem einzigen Reaktionsbehälter befinden.

3. Verfahren nach Anspruch 1 oder 2, wobei der Volumenanteil der wässrigen Phase in der Reaktionszone wenigstens 70 %, besonders bevorzugt wenigstens 80 %, bezogen auf das Gesamtvolumen des zweiphasigen Reaktionsgemischs in der Reaktionszone beträgt.

4. Verfahren nach Anspruch 1, wobei zur Rückvermischung wenigstens ein der Reaktionszone zugeführter Strom und/oder wenigstens ein in einem externen Kreislauf geführter Strom aus der Reaktionszone (Wälzstrom) eingesetzt wird.

5. Verfahren nach einem der vorhergehenden Ansprüche, wobei die Reaktionszone als Schlaufenreaktor oder Rührkessel ausgeführt ist.

6. Verfahren nach einem der vorhergehenden Ansprüche, wobei die Reaktionszone als Freistrahlreaktor ausgeführt ist.

7. Verfahren nach einem der vorhergehenden Ansprüche, wobei die Zuführung des Aldehyds im oberen Bereich der Reaktionszone erfolgt.

8. Verfahren nach einem der vorhergehenden Ansprüche, wobei zur Erzeugung der in der kontinuierlichen wässrigen katalysatorhaltigen Phase dispergierten aldehydhaltigen Phase in der Reaktionszone wenigstens eine Mischdüse eingesetzt wird.

9. Verfahren nach Anspruch 8, wobei die in den Reaktor insgesamt eingetragene Mischleistung höchstens 0,5 kW pro m³ Flüssigvolumen, bevorzugt höchstens 0,3 kW pro m³ Flüssigvolumen, beträgt.

10. Verfahren nach einem der vorhergehenden Ansprüche, wobei die Zuführung des Aldehyds über eine Zweistoffdüse erfolgt.

11. Verfahren nach Anspruch 10, wobei in die Zweistoffdüse der Aldehyd und ein in einem externen Kreislauf geführter Strom aus der Reaktionszone (Wälzstrom) geführt werden.

12. Verfahren nach einem der vorhergehenden Ansprüche, wobei die Beruhigungszone und die Reaktionszone rohrförmig ausgestaltet sind und die Beruhigungszone einen größeren Durchmesser aufweist als die Reaktionszone.

13. Verfahren nach einem der vorhergehenden Ansprüche, wobei aus der kontinuierlichen organischen Phase im oberen Bereich der Beruhigungszone ein Austrag entnommen wird.

14. Verfahren nach Anspruch 13, wobei der Austrag aus der Beruhigungszone einer weiteren Umsetzung in wenigstens einem Reaktor unterzogen wird.

15. Verwendung einer Vorrichtung, umfassend einen Reaktor, der eine Reaktionszone und eine unmittelbar darüber befindliche Beruhigungszone aufweist, wobei die Reaktionszone eine Vorrichtung zur Erzeugung einer in einer kontinuierlichen wässrigen Phase dispergierten organischen Phase aufweist und die Beruhigungszone die Koaleszenz des zweiphasigen Reaktionsgemischs und die Ausbildung einer kontinuierlichen organischen Phase im oberen Bereich der Beruhigungszone ermöglicht, zur katalytischen Aldolkondensation wenigstens eines Aldehyds in einem zweiphasigen flüssigen Reaktionsgemisch.

## Claims

1. A process for the catalytic aldol condensation of at least one aldehyde in a two-phase liquid reaction mixture in a reactor which has a reaction zone and a disengagement zone located directly above the reaction zone, wherein the reaction zone is backmixed and an aldehyde-comprising phase dispersed in a continuous aqueous catalyst-comprising phase is produced in the reaction zone and a stream of the two-phase reaction mixture is allowed to rise from the reaction zone into the disengagement zone and coalesce, with a continuous organic phase being formed in the upper region of the disengagement zone.

2. The process according to claim 1, wherein the reaction zone and the disengagement zone are located in a single reaction vessel.

3. The process according to claim 1 or 2, wherein the proportion by volume of the aqueous phase in the reaction zone is at least 70%, particularly preferably at least 80%, based on the total volume of the two-phase reaction mixture in the reaction zone.

4. The process according to claim 1, wherein at least one stream fed into the reaction zone and/or at least one stream from the reaction zone conveyed in an external circuit (circulation stream) is/are used for backmixing.

5. The process according to any of the preceding claims, wherein the reaction zone is configured as a loop reactor or stirred vessel.

6. The process according to any of the preceding claims, wherein the reaction zone is configured as a free jet reactor.

7. The process according to any of the preceding claims, wherein the aldehyde is introduced in the upper region of the reaction zone.

8. The process according to any of the preceding claims, wherein at least one mixing nozzle is used to produce the aldehyde-comprising phase dispersed in the continuous aqueous catalyst-comprising phase in the reaction zone.

9. The process according to claim 8, wherein the total mixing power introduced into the reactor is not more than 0.5 kW per m³ of liquid volume, preferably not more than 0.3 kW per m³ of liquid volume.

10. The process according to any of the preceding claims, wherein the aldehyde is introduced via a two-fluid nozzle.

11. The process according to claim 10, wherein the aldehyde and a stream from the reaction zone conveyed in an external circuit (circulation stream) are introduced into the two-fluid nozzle.

12. The process according to any of the preceding claims, wherein the disengagement zone and the reaction zone are tubular and the disengagement zone has a greater diameter than the reaction zone.

13. The process according to any of the preceding claims, wherein a discharge is taken off from the continuous organic phase in the upper region of the disengagement zone.

14. The process according to claim 13, wherein the discharge from the disengagement zone is subjected to a further reaction in at least one reactor.

15. The use of an apparatus comprising a reactor which has a reaction zone and a disengagement zone located directly above the reaction zone, wherein the reaction zone has a device for producing an organic phase dispersed in a continuous aqueous phase and the disengagement zone allows the coalescence of the two-phase reaction mixture and the formation of a continuous organic phase in the upper region of the disengagement zone, for the catalytic aldol condensation of at least one aldehyde in a two-phase liquid reaction mixture.

## Revendications

1. Procédé pour la condensation aldol catalytique d'au moins un aldéhyde dans un mélange réactionnel liquide à deux phases dans un réacteur, qui présente une zone de réaction et une zone de repos se trouvant immédiatement au-dessus de celle-ci, la zone de réaction étant mélangée en retour et dans lequel on produit une phase contenant un aldéhyde dispersée dans une phase aqueuse continue contenant un catalyseur dans la zone de réaction et on laisse monter un flux du mélange réactionnel à deux phases de la zone de réaction dans la zone de repos et on le laisse coalescer, une phase organique continue se formant dans la partie supérieure de la zone de repos.

2. Procédé selon la revendication 1, la zone de réaction et la zone de repos se trouvant dans un seul récipient de réaction.

3. Procédé selon la revendication 1 ou 2, la proportion volumique de la phase aqueuse dans la zone de réaction représentant au moins 70%, de manière particulièrement préférée au moins 80%, par rapport au volume total du mélange réactionnel à deux phases dans la zone de réaction.

4. Procédé selon la revendication 1, au moins un flux introduit dans la zone de réaction et/ou au moins un flux guidé dans un circuit externe sortant de la zone de réaction (flux en circulation) étant utilisé(s) pour le mélange en retour.

5. Procédé selon l'une quelconque des revendications précédentes, la zone de réaction étant réalisée sous forme de réacteur à boucle à circulation interne ou de cuve agitée.

6. Procédé selon l'une quelconque des revendications précédentes, la zone de réaction étant réalisée sous forme de réacteur à jet libre.

7. Procédé selon l'une quelconque des revendications précédentes, l'introduction de l'aldéhyde ayant lieu dans la partie supérieure de la zone de réaction.

8. Procédé selon l'une quelconque des revendications précédentes, au moins une buse de mélange étant utilisée pour la production de la phase contenant un aldéhyde dispersée dans la phase aqueuse continue, contenant un catalyseur dans la zone de réaction.

9. Procédé selon la revendication 8, la puissance de mélange introduite au total dans le réacteur étant d'au plus 0,5 kW par m³ de volume de liquide, de préférence d'au plus 0,3 kW par m³ de volume de liquide.

10. Procédé selon l'une quelconque des revendications précédentes, l'alimentation en aldéhyde ayant lieu via une buse à deux substances.

11. Procédé selon la revendication 10, l'aldéhyde et un flux guidé dans un circuit externe sortant de la zone de réaction (flux en circulation) étant guidés dans la buse à deux substances.

12. Procédé selon l'une quelconque des revendications précédentes, la zone de repos et la zone de réaction étant réalisées sous forme tubulaire et la zone de repos présentant un diamètre supérieur à celui de la zone de réaction.

13. Procédé selon l'une quelconque des revendications précédentes, un produit étant prélevé de la phase organique continue dans la partie supérieure de la zone de repos.

14. Procédé selon la revendication 13, le produit sortant de la zone de repos étant soumis à une autre transformation dans au moins un réacteur.

15. Utilisation d'un dispositif comprenant un réacteur, qui présente une zone de réaction et une zone de repos se trouvant immédiatement au-dessus de celle-ci, la zone de réaction présentant un dispositif pour la production d'une phase organique dispersée dans une phase aqueuse continue et la zone de repos permettant la coalescence du mélange réactionnel à deux phases et la formation d'une phase organique continue dans la partie supérieure de la zone de repos, pour la condensation aldol catalytique d'au moins un aldéhyde dans un mélange réactionnel liquide à deux phases.
